(19) Europäisches Patentamt / European Patent Office / Office européen des brevets

(11) **EP 2 271 367 B1**

(12) **EUROPEAN PATENT SPECIFICATION**

(45) Date of publication and mention
of the grant of the patent:
**18.05.2016 Bulletin 2016/20**

(51) Int Cl.:
*A61K 36/37* (2006.01)    *A61K 45/00* (2006.01)
*A61K 36/82* (2006.01)    *A61P 7/06* (2006.01)
*A61K 36/45* (2006.01)    *A61K 31/7008* (2006.01)

(21) Application number: **09726975.7**

(22) Date of filing: **03.04.2009**

(86) International application number:
**PCT/JP2009/057300**

(87) International publication number:
**WO 2009/123364 (08.10.2009 Gazette 2009/41)**

(54) **MINERAL ABSORPTION ACCELERATOR AND IRON DEFICIENCY ANEMIA IMPROVER OR FOOD COMPOSITION**

MINERALAUFNAHMEBESCHLEUNIGER UND MITTEL ZUR VERBESSERUNG VON EISENMANGELANÄMIE ODER NAHRUNGSMITTELZUSAMMENSETZUNG

ACCÉLÉRATEUR D'ABSORPTION DE MINÉRAUX ET AGENT OU COMPOSITION ALIMENTAIRE AMÉLIORANT L'ANÉMIE FERRIPRIVE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **03.04.2008 JP 2008097427**

(43) Date of publication of application:
**12.01.2011 Bulletin 2011/02**

(73) Proprietor: **FUJIFILM Corporation
Tokyo 106-0031 (JP)**

(72) Inventor: **UEDA, Fumitaka
Kanagawa (JP)**

(74) Representative: **Hoffmann Eitle
Patent- und Rechtsanwälte PartmbB
Arabellastraße 30
81925 München (DE)**

(56) References cited:
EP-A1- 1 645 557       JP-A- 2002 010 752
JP-A- 2002 332 236     JP-A- 2004 154 126
JP-A- 2004 175 790     JP-A- 2004 194 635

JP-A- 2007 195 510

• DATABASE BIOSIS [Online] BIOSCIENCES INFORMATION SERVICE, PHILADELPHIA, PA, US; April 2007 (2007-04), MONARREZ-ESPINO JOEL ET AL: "Randomized trial of guava as a source of ascorbic acid to reduce iron deficiency in indigenous schoolchildren of Mexico", XP009150979, Database accession no. PREV200700335265 & FASEB JOURNAL, vol. 21, no. 5, April 2007 (2007-04), page A682, EXPERIMENTAL BIOLOGY 2007 ANNUAL MEETING; WASHINGTON, DC, USA; APRIL 28 -MAY 02, 2007 ISSN: 0892-6638
• DATABASE WPI Week 200751 Thomson Scientific, London, GB; AN 2007-520025 XP002655809, & JP 2007 161620 A (TAISHO PHARM CO LTD) 28 June 2007 (2007-06-28)
• FAILLA,M.L. ET AL.: 'The absorption and retention of dietary zinc by type I diabetic rats are increased by chronic treatment with acarbose' DRUGS IN DEVELOPMENT vol. 1, 1993, pages 155 - 62, XP008153911
• YOO,W.H. ET AL.: 'Marked weight loss in a type 2 diabetic patient treated with acarbose' DIABETES CARE vol. 22, no. 4, 1999, pages 645 - 6, XP008143279
• HOLLANDER,P. ET AL.: 'Safety Profile of Acarbose, an a-Glucosidase Inhibitor' DRUGS vol. 44, no. SUP.3, 1992, pages 47 - 53, XP008143282

**Description**

Technical Field

This invention relates to a mineral absorption accelerator and an iron deficiency anemia improver or a food composition, which comprises a component having α-glucosidase inhibitory action or a pulverized product or extract of a plant of the genus *Salacia* for use in a method of preventing or improving anemia as defined in the claims.

Background Art

In recent years, the food situation of the Japanese people has been improved to such a level that the average ingestion quantity of almost all nutrient substances satisfies the necessary nutritive quantity, but the ingestion quantity of iron, calcium, zinc, copper and the like minerals does not satisfy necessary levels (National Nutrition Survey, 2001).

Particularly in the case of iron, there is a possibility that when its insufficient ingestion continues, it causes iron deficiency anemia via a latent iron deficiency which does not show anemic symptoms. It is said that this iron deficiency anemia is frequently found mainly in adult females and about half of the adult females are suffering from the latent iron deficiency or iron deficiency anemia.

In order to supplement the iron content run out in the body, it is necessary to ingest iron in an amount larger than the daily requirement or more, but since iron is poorly absorbable, a method for increasing absorption rate of the mineral is efficient rather than increasing its ingesting amount. Accordingly, great concern has been directed toward a food having high iron absorbability and a substance capable of accelerating absorption of iron, and 1 mg or more but 200 mg or less of iron is preferable as its ingesting amount for one day. On the other hand, various pharmaceutical preparations have been devised as the pharmaceutical preparations of iron compounds to be used for the prevention or treatment of anemia, but side effects for digestive organs caused by iron, such as loss of appetite, nausea, vomiting, abdominal pain, constipation, diarrhea and the like, are causing clinical problems.

So far, a finding that restoration of hemoglobin is accelerated when an iron preparation and proline are concomitantly administered to an anemia model rat (J. Nutr. Sci. Vitaminol., 49, 7 - 12, 2003), a method which uses yeast cell wall polysaccharides (JP-A-2002-255832), a method which concomitantly use a group of isoleucine, valine, leucine and arginine and an iron preparation (JP-A-2008-50277) and the like have been reported, and these have the effect to improve absorption of iron but do not improve the above-mentioned side effects. The problem of iron deficiency is also considered by Monarrez- Espino, J. et.al., FASEB Journal, (2007), 21, p A682, Failla, M.L. et.al., Drugs in Development, 1993, 1,p 155-162 and in JP2004154126, JP2004194635, JP2007161620 and EP1645557.

Disclosure of the Invention

Accordingly, the invention aims at providing a food, a drink, a feed and a pharmaceutical composition, which are free from side effects, have high safety and are possessed of the actions to accelerate mineral absorption and to prevent or improve anemia.

During the process of carrying out a study on components having α-glucosidase inhibitory activity, the present inventors have found that these components have the effect to accelerate absorption of iron. This effect is a finding which has been completely unknown until now.

Thus, in order to solve the above-mentioned problems, a mineral absorption accelerating agent or anemia improving agent, which has no side effect and increases utilizing efficiency of minerals including iron, has been accomplished in the invention, by making use of a component having α-glucosidase inhibitory activity.

The invention includes the following constructions.

(1) An α-glucosidase inhibitory component which is at least one selected from the group consisting of acarbose, voglibose, miglitol, a pulverized product or extract of a plant of the genus Salacia, deoxynojirimycin, a guava leaf polyphenol, a mulberry leaf extract, a green tea extract and a fermented black beans extract for use in a method of preventing or improving anemia.

(2) The α-glucosidase inhibitory component as described in (1) above, wherein the α-glucosidase inhibitory component is at least one selected from the group consisting of salacinol and kotalanol.

(3) The α-glucosidase inhibitory component as described in either (1) or (2) above, which accelerates absorption of iron, calcium, magnesium or zinc.

(4) The α-glucosidase inhibitory component as described in any one of (1) to (3) above, which shows an activity as a sucrase 50% inhibition concentration (IC$_{50}$ value) of 0.01 μg/ml or more but 800 μg/ml or less.

(5) The agent for improving anemia as described in (4) above, which is an agent for improving iron deficiency, the agent further comprising:

a compound equivalent to 1 mg or more of iron as ingesting amount for one day.

Best Mode For Carrying Out the Invention

The $\alpha$-glucosidase inhibitory component to be used in the invention is not particularly limited, with the proviso that it is a component which inhibits the $\alpha$-glucosidase that is present in the small intestinal epithelium.

Particularly preferred as the $\alpha$-glucosidase inhibitory component is salacinol or kotalanol. It is desirable that these are contained as a pulverized product or extract of a plant of the genus *Salacia,* but these may also be chemically synthesized products.

The above described examples of the $\alpha$-glucosidase inhibitory component may be used singly or in combination thereof.

The $\alpha$-glucosidase inhibitory component of the invention has a sucrase 50% inhibition concentration ($IC_{50}$ value) of preferably 0.01 $\mu$g/ml or more but 800 $\mu$g/ml or less. Also, the sucrase 50% inhibition concentration is more preferably 0.1 $\mu$g/ml or more but 600 $\mu$g/ml or less, further preferably 0.5 $\mu$g/ml or more but 450 $\mu$g/ml or less.

The sucrase 50% inhibition concentration ($IC_{50}$ value) is measured by the following method.

[Test method 1] Measurement of sucrase $IC_{50}$ value

Preparation of sample solution: A 2 mg portion of a sample is weighed, put into a tube and suspended by adding 2 ml of water thereto, thereby preparing a sample solution having a concentration of 1 mg/ml. This is diluted with water to 0, 50, 100, 250 or 500 $\mu$g/ml.

Preparation of substrate liquid: Sucrose is dissolved in 0.2 M maleate buffer (pH 6.0) to a sucrose concentration of 100 mM, and this is used as the substrate liquid.

Preparation of crude enzyme liquid: A 1 g portion of intestinal acetone powder rat (mfd. by SIGMA) is suspended in 10 ml of physiological saline and then centrifuged (3,000 rpm, 4°C, 5 min). The thus obtained supernatant is separated and used as the crude enzyme liquid.

A 400 $\mu$l portion of the substrate liquid is added to 500 $\mu$l of each of the aforementioned sample solutions having respective concentrations and preliminarily heated at 37°C for 5 minutes in a water bath. A 100 $\mu$l portion of the crude enzyme liquid is added to each of them and allowed to undergo the reaction at 37°C for 60 minutes. After completion of the reaction, the reaction is terminated by deactivating the enzyme through heating at 95°C for 2 minutes. Determination of concentration of the thus formed glucose is carried out using a commercially available kit for mutarotase glucose oxidase method (Glucose CII Test Wako, mfd. by Wako Pure Chemical Industries).

Preparation of blank: A 200 $\mu$l portion of the substrate liquid and 50 $\mu$l of the crude enzyme liquid are added to 250 $\mu$l of each of the aforementioned sample solutions having respective concentrations and immediately heated at 95°C for 2 minutes to effect thermal deactivation of the enzyme, to be used as blank data.

By preparing a calibration curve from the thus obtained values, the concentration which inhibits 50% of the enzyme activity ($IC_{50}$ value) is calculated.

It is desirable that the $\alpha$-glucosidase inhibitory component of the invention accelerates absorption of iron, calcium, magnesium or zinc.

Also, it is desirable that the agent for improving anemia, which comprises the $\alpha$-glucosidase inhibitory component of the invention, is an agent for improving iron deficiency and further comprises a compound equivalent to 1 mg or more of iron as ingesting amount for one day. Examples of the compound include sodium ferrous citrate, ferric pyrophosphate, ferrous fumarate, ferrous chloride and heme iron.

In addition, it is desirable that the $\alpha$-glucosidase inhibitory component of the invention is contained in food and drink or food and drink materials.

The $\alpha$-glucosidase inhibitory component and anemia improving agent of the invention may be made into a drink, a liquid food such as yogurt, a jelly type food, a powdery food material and the like shapes or into tablets, hard capsules, soft capsules or granules. In the latter case, crystalline cellulose, magnesium stearate and the like fillers and corn starch, alginic acid and the like swelling agents can be used. In addition, shellac or sugar, a film coating base material, YeastWrap and the like can be used as the coating agent of tablets, capsules or granules.

The plant of the genus *Salacia* of the invention is a plant of the family Celastraceae growing wild mainly in Sri Lanka, India and Southeast Asia regions, and more illustratively, one species or more of plants selected from *Salacia reticulata, S. oblonga, S. prinoides* and *S. chinensis* are used. Pulverized products of these plants and extract powders extracted from roots, stems, leaves, flowers, fruits and the like edible parts thereof are used. One or more of these parts may be used by mixing them. More preferably, extract powders extracted from roots or stems are used.

In the case of extract powders of a plant of the genus *Salacia,* those which are obtained by a solvent extraction from the aforementioned edible parts are dried and used. As the extraction solvent, it may be selected from water, alcohols including methanol and ethanol and mixed solvents of water and alcohols or ketones such as acetone. Preferably, water, an alcohol or a hydrous alcohol is used. More preferably, hot water, ethanol or hydrous ethanol is used as the extraction solvent. Regarding the alcohol concentration of the aforementioned hydrous alcohol, those having a concentration of from 30 to 90%, preferably from 40 to 70% may be used.

Spray drying, freeze drying and the like can be cited as the drying method.

According to the invention, in order to improve periodical discoloration of the extraction extract powder of a plant of the genus *Salacia,* it is desirable to contain therein calcium carbonate or silicon dioxide in an amount of 1% or more of the weight when made into a shape of a tablet or hard capsule. In addition, it is possible to use a low moisture absorption material or moisture absorbent which can be used as a food or food additive. Preferably, cellulose, crystalline cellulose, powder cellulose, microcrystalline cellulose, lactose, an oligosaccharide, a sugar alcohol, trehalose, magnesium stearate, calcium stearate and the like are used as the low moisture absorption material. As the moisture absorbent, silicic acid salts, magnesium carbonate, a ferrocyanide, polysaccharides and the like are used. More preferably, crystalline cellulose, microcrystalline cellulose or lactose is used as the low moisture absorption material.

The term mineral as used herein means iron, calcium, magnesium, sodium, potassium, phosphorus, manganese, copper, zinc, molybdenum, manganese, cobalt, selenium, iodide, fluorine and the like which are required in the living body.

As the materials of calcium and magnesium, for example, dolomite can be cited, but there may be exemplified all of the natural calcium which can be used in food, such as egg shell calcium, coral calcium, sea urchin shell calcium, fossilized marine algal calcium, pearl calcium, cattle bone calcium, shell calcium, fish bone dust calcium, fish scale calcium, milk calcium and the like.

As the dolomite, a commercially available article obtained by pulverizing dolomite raw ore after its crude crushing and heat sterilization can be used in general.

Iron can be ingested from heme iron, iron yeast and the like. The heme iron occupies about 40% of the iron contained in meats, fishes and internal organs, and its intestinal absorption is good in comparison with non-heme iron. The heme iron is generally obtained by treating hemoglobin with an enzyme and then subjecting it to ultrafiltration or isoelectric precipitation and subsequent drying, but a commercially available article can also be used.

As the marine algae from which iodine can be obtained, brown algae, red algae and the like or a mixture thereof can be used, and these can be blended as dried products or pulverized products. Such dried products and their pulverized products are commercially available.

The term yeasts means yeast in which a mineral (manganese, copper, molybdenum, iron, magnesium, zinc, selenium, chromium, iodine or the like) is accumulated into the cell by culturing the yeast using a medium to which the mineral is added in a high concentration. These are obtained via concentration, sterilization, drying and the like steps after culturing the yeast using a medium supplemented with a mineral and collecting the cells, and those which are on the market can be used.

As the yeast, baker's yeast and beer yeast are generally used widely. Illustratively, manganese yeast, copper yeast, molybdenum yeast, iron yeast, magnesium yeast, zinc yeast, selenium yeast, chromium yeast, iodine yeast and the like can be exemplified.

A compound necessary for forming into the powders, solid preparations or liquid preparations of the invention, and the like may be optionally included. As examples of such a compound, erythritol, maltitol, hydroxypropyl cellulose, kaolin, talk and the like can be cited.

The invention is described in the following using examples.

[EXAMPLES]

<Example 1>

Root and stem parts of *Salacia reticulata* and *Salacia oblonga* were pulverized and then subjected to a hot water extraction process, and the thus obtained liquid was spraydried to obtain a Salacia extract powder.

Also, 300 g of dry powder of mulberry leaves was extracted by adding 1 liter of 25% ethanol and the filtrate was dried by removing the solvent under a reduced pressure, thereby obtaining a mulberry leaf extract.

Dry powder of guava leaves was extracted by adding hot water of 95°C and the filtrate was dried by removing the solvent under a reduced pressure, thereby obtaining a guava leaf extract.

Powders having the formulations shown in Table 1 were prepared using the thus obtained extract powders, and their sucrase IC50 values were measured by the method described in [Test method 1].

In addition, by subjecting the formulated powders of Table 1 to tablet making, tablets of the sample 1 to sample 13 were prepared.

Table 1

| | Salacia extract powder | Mulberry leaf extract | Guava leaf extract | Green tea extract | Ferric pyrophosphate | Crystalline cellulose | Sucrase IC$_{50}$ value (μg/ml) | |
|---|---|---|---|---|---|---|---|---|
| α-Glucosidase inhibitory component formulation example and sucrase IC$_{50}$ value | | | | | | | | |
| Sample 1 | 0 mg | 0 mg | 0 mg | 0 mg | 0 mg | 250 mg | 2000 or more | Comparative example |
| Sample 2 | 0 mg | 0 mg | 0 mg | 0 mg | 5 mg | 250 mg | 2000 or more | Comparative example |
| Sample 3 | 25 mg | 0 mg | 0 mg | 0 mg | 0 mg | 225 mg | 600 | Inventive |
| Sample 4 | 50 mg | 0 mg | 0 mg | 0 mg | 0 mg | 200 mg | 280 | Inventive |
| Sample 5 | 200 mg | 0 mg | 0 mg | 0 mg | 0 mg | 50 mg | 65 | Inventive |
| Sample 6 | 0 mg | 100 mg | 0 mg | 0 mg | 0 mg | 150 mg | 102 | Inventive |
| Sample 7 | 50 mg | 0 mg | 0 mg | 50 mg | 0 mg | 150 mg | 200 | Inventive |
| Sample 8 | 0 mg | 0 mg | 100 mg | 0 mg | 0 mg | 150 mg | 232 | Inventive |
| Sample 9 | 0 mg | 100 mg | 0 mg | 50 mg | 0 mg | 100 mg | 75 | Inventive |
| Sample 10 | 50 mg | 0 mg | 0 mg | 0 mg | 5 mg | 200 mg | 280 | Inventive |
| Sample 11 | 0 mg | 100 mg | 0 mg | 0 mg | 5 ng | 150 mg | 104 | Inventive |
| Sample 12 | 50 mg | 0 mg | 0 mg | 50 mg | 5 mg | 150 mg | 204 | Inventive |
| Sample 13 | 0 mg | 0 mg | 100 mg | 0 mg | 5 mg | 150 mg | 230 | Inventive |
| * Green tea extract: Sunfenon 100s, mfd. by Taiyo Kagaku was used. | | | | | | | | |

Each group consisting of five adult women who answered a questionnaire carried out in advance that they have an anemic tendency was asked to orally ingest one tablet of the samples 1 to 13, respectively before meals every day, and this was repeated 30 days. Blood samples were collected on the day before the commencement of the ingestion and on the next day of the completion of the ingestion, and the number of red blood cells, hemoglobin, hematocrit and the amount of serum iron were measured. The results are shown in Table 2. Each value is average value of five volunteers, and relative values when the value of sample 1 was regarded as 100 are shown by Table 2.

Table 2

| Effect of ingestion of samples 1 to 13 | | | | | |
|---|---|---|---|---|---|
| | Red blood cell count | Hematocrit | Hemoglobin | Serum iron | |
| Sample 1 | 100 | 100 | 100 | 100 | Comparative example |
| Sample 2 | 102 | 101 | 102 | 102 | Comparative example |
| Sample 3 | 103 | 103 | 104 | 104 | Inventive |
| Sample 4 | 110 | 111 | 110 | 109 | Inventive |
| Sample 5 | 113 | 112 | 112 | 112 | Inventive |
| Sample 6 | 109 | 108 | 109 | 108 | Inventive |
| Sample 7 | 113 | 114 | 113 | 113 | Inventive |
| Sample 8 | 107 | 106 | 106 | 106 | Inventive |
| Sample 9 | 112 | 112 | 111 | 110 | Inventive |
| Sample 10 | 114 | 115 | 114 | 114 | Inventive |
| Sample 11 | 113 | 112 | 112 | 111 | Inventive |
| Sample 12 | 116 | 115 | 116 | 115 | Inventive |
| Sample 13 | 112 | 111 | 112 | 112 | Inventive |

Since the values of red blood cell count, hematocrit and serum iron significantly increased by the ingestion of the samples of the invention, it was revealed that absorption of iron is improved by the ingestion of the samples of the invention. It was found from the results of samples 3 to 9 that ingestion of even the component of the invention alone increases absorbed amount of iron obtained from everyday meals and therefore has the effect to improve anemia. In addition, it was found that the samples 10 to 13 to which iron pyrophosphate was added have particularly high effect to improve iron deficiency anemia.

According to the questionnaires carried out during the ingestion period, there was no one who complained of an unpleasant feeling in the stomach and intestines by the ingestion of the component of the invention, so that it was revealed also that the agent of the invention has a characteristic of being easily drinkable without unpleasant feeling in comparison with the conventional iron preparations.

<Example 2>

Salacinol was isolated from the Salacia extract powder prepared in Example 1 by the method of Yoshikawa described in Bioorganic & Medicinal Chemistry, 10 (2002) 1547 - 1554. Using this and $\alpha$-glucosidase inhibitory agents, voglibose and acarbose, the following test was carried out.

SD rats (males) of 4 weeks of age after birth were preliminarily reared for 1 week and then divided into a control group and 6 sample ingestion groups, each group consisting of 6 animals, at random based on body weight. In the rats of salacinol administration group, 0.1% salacinol aqueous solution was orally administered at a dose of 2.0 g/day per unit weight of rat. In the rats of voglibose administration group, 0.4% voglibose aqueous solution was orally administered at a dose of 2.0 g/day per unit weight of rat. In the rats of acarbose administration group, 0.4% acarbose aqueous solution was orally administered at a dose of 2.0 g/day per unit weight of rat. In the rats of Salacia extract powder administration group, 1% Salacia extract powder aqueous solution was orally administered at a dose of 2.0 g/day per unit weight of rat. In the rats of mulberry extract powder administration group, 1% mulberry extract powder aqueous solution was orally administered at a dose of 2.0 g/day per unit weight of rat. In the rats of Salacia extract powder + green tea extract administration group, 1% Salacia extract powder + 1% green tea extract mixed aqueous solution was orally administered at a dose of 2.0 g/day per unit weight of rat. In the rats of control group, water was orally administered at a dose of 2.0 g/day per unit weight of rat.

Each rat was reared with a solid feed (CRF-1 mfd. by Oriental Yeast) for 14 days and then transferred into a cage for metabolism analysis, and urine and feces were collected for a period of 4 days to measure concentrations of iron, calcium, magnesium and zinc. Detection of minerals from feces was carried out using inductively coupled plasma emission spectrometry, and those from urine and serum using atomic absorption method. Absorption rate and internal holding rate of each mineral were calculated using the following formulae.

$$\texttt{Absorbed amount = ingested amount - discharged}$$
$$\texttt{amount into feces}$$

$$\texttt{Absorption rate (\%) = absorbed amount \div ingested}$$
$$\texttt{amount x 100}$$

$$\texttt{Internally retained amount = absorbed amount -}$$
$$\texttt{discharged amount into urine}$$

$$\texttt{Internal retention rate (\%) = internally retained}$$
$$\texttt{amount \div ingested amount x 100}$$

Results of the absorption rate and internal retention rate of minerals and concentration of mineral in serum by the mineral balance test are respectively shown in Tables 3 to 5.

Table 3

| Absorption rate of minerals | | | | |
|---|---|---|---|---|
| | Absorption rate of iron (%) | Absorption rate of calcium (%) | Absorption rate of magnesium (%) | Absorption rate of zinc (%) |
| Control group | 24.8 | 21.0 | 10.8 | 7.2 |
| Salacinol ingestion group | 38.5 | 33.2 | 20.5 | 30.1 |
| Voglibose ingestion group | 37.8 | 32.5 | 21.2 | 29.8 |
| Acarbose ingestion group | 37.5 | 32.8 | 20.8 | 29.3 |
| Salacia extract powder ingestion group | 33.4 | 29.3 | 18.5 | 28.4 |
| Mulberry extract powder ingestion group | 31.2 | 28.2 | 18.2 | 27.2 |
| Salacia + green tea ingestion group | 35.0 | 31.4 | 20.4 | 30.7 |

Table 4

| Internal retention rate of minerals | | | | |
|---|---|---|---|---|
| | Internal retention rate of iron (%) | Internal retention rate of calcium (%) | Internal retention rate of magnesium (%) | Internal retention rate of zinc (%) |
| Control group | 21.4 | 20.1 | 10.1 | 5.8 |
| Salacinol ingestion group | 34.2 | 32.3 | 23.6 | 19.5 |
| Voglibose ingestion group | 33.9 | 32.1 | 24.0 | 18.6 |
| Acarbose ingestion group | 32.8 | 31.5 | 23.1 | 18.4 |
| Salacia extract powder ingestion group | 32.5 | 30.8 | 21.6 | 18.4 |
| Mulberry extract powder ingestion group | 30.4 | 29.1 | 20.7 | 17.2 |
| Salacia + green tea ingestion group | 33.6 | 32.4 | 22.9 | 19.9 |

Table 5

| Concentration of minerals in serum | | | | |
|---|---|---|---|---|
| | Concentration of iron ($\mu$g/dl) | Concentration of calcium (mg/dl) | Concentration of magnesium (mg/dl) | Concentration of zinc ($\mu$g/dl) |
| Control group | 184.5 | 10.2 | 1.85 | 106.8 |
| Salacinol ingestion group | 298.6 | 11.8 | 2.06 | 122.7 |
| Voglibose ingestion group | 292.5 | 10.9 | 2.04 | 120.2 |
| Acarbose ingestion group | 289.3 | 10.9 | 2.01 | 121.3 |
| Salacia extract powder ingestion group | 288.4 | 10.8 | 1.98 | 118.8 |
| Mulberry extract powder ingestion group | 276.1 | 10.8 | 1.98 | 118.2 |
| Salacia + green tea ingestion group | 296.8 | 11.1 | 2.04 | 120.5 |

It was revealed that absorption of iron, calcium, magnesium, zinc and the like minerals is sharply increased by the ingestion of the component of the invention, in comparison with the control group. Accordingly, it is considered that poor physical conditions and diseases accompanied by insufficient minerals can be prevented by the ingestion of the component of the invention.

(Example 3)

Preparation of tablets using *Salacia* extract powder By preparing tablets using the formulation shown in Table 6, a supplement to which shellac coating was applied was prepared.

Table 6

| Tablet formulation example using the *Salacia* extract powder of the invention | |
|---|---|
| Raw material name | Blending amount (wt%) |
| *Salacia* extract powder | 25.0 |
| Red wine polyphenol | 10.0 |
| Onion outer skin extract powder | 6.0 |
| Green tea extract | 15.0 |
| Hematococcus algal pigment | 1.0 |
| Chrome yeast | 4.0 |
| Iron pyrophosphate | 10.0 |
| Crystalline cellulose | 23.0 |
| Sucrose fatty acid ester | 2.0 |
| Lactose | 1.0 |
| Calcium carbonate | 1.0 |
| Atomized silicon dioxide | 2.0 |

The effect shown by Example 1 was obtained by the ingestion of tablets of this formulation. In addition, "physical conditions became lighter", "fatigue became less" and the like reports were obtained from the ingested volunteers.

Industrial Applicability

According to the invention, there is provided a food, a drink, a feed and a pharmaceutical composition, which are free from side effects, have high safety and are possessed of the actions to accelerate mineral absorption and to prevent or improve anemia.

**Claims**

1. An $\alpha$-glucosidase inhibitory component which is at least one selected from the group consisting of acarbose, voglibose, miglitol, a pulverized product or extract of a plant of the genus Salacia, deoxynojirimycin, a guava leaf polyphenol, a mulberry leaf extract, a green tea extract and a fermented black beans extract for use in a method of preventing or improving anemia.

2. The $\alpha$-glucosidase inhibitory component for use according to claim 1, wherein the $\alpha$-glucosidase inhibitory component is at least one selected from the group consisting of salacinol and kotalanol.

3. The $\alpha$-glucosidase inhibitory component for use according to either claim 1 or claim 2, which accelerates absorption of iron, calcium, magnesium or zinc.

4. The $\alpha$-glucosidase inhibitory component for use according to any one of claims 1 to 3, which shows an activity as a sucrase 50% inhibition concentration ($IC_{50}$ value) of 0.01 $\mu$g/ml or more but 800 $\mu$g/ml or less.

5. The agent for improving anemia for use according to claim 4, which is an agent for improving iron deficiency, the agent further comprising: a compound equivalent to 1 mg or more of iron as ingesting amount for one day.

6. The $\alpha$-glucosidase inhibitory component for use according to any one of claims 1 to 5 which is contained in a food or drink material.

**Patentansprüche**

1. α-Glucosidase-inhibierende Komponente, die zumindest eine ist, die ausgewählt ist aus der Gruppe bestehend aus Acarbose, Voglibose, Miglitol, einem pulverisierten Produkt oder Extrakt einer Pflanze der Gattung Salacia, Deoxynojirimycin, einem Guavenblatt-Polyphenol, einem Maulbeerbaumblattextrakt, einem Grüntee-Extrakt und einem Extrakt fermentierter schwarzer Bohnen, zur Verwendung in einem Verfahren zur Vorbeugung oder Verbesserung von Anämie.

2. α-Glucosidase-inhibierende Komponente zur Verwendung gemäß Anspruch 1, worin die α-Glucosidase-inhibierende Komponente mindestens eine ist, die ausgewählt ist aus der Gruppe, bestehend aus Salacinol und Kotalanol.

3. α-Glucosidase-inhibierende Komponente zur Verwendung gemäß Anspruch 1 oder 2, welche die Absorption von Eisen, Calcium, Magnesium oder Zink beschleunigt.

4. α-Glucosidase-inhibierende Komponente zur Verwendung gemäß irgendeinem der Ansprüche 1 bis 3, welche eine Aktivität als Sucrase-50 %-Inhibierungskonzentration ($IC_{50}$)-Wert von 0,01 µg/ml oder mehr, jedoch 800 µg/ml oder weniger zeigt.

5. Mittel zur Verbesserung von Anämie zur Verwendung gemäß Anspruch 4, welches ein Mittel zur Verbesserung eines Eisenmangels ist, wobei das Mittel ferner umfasst:

   eine Verbindung, die 1 mg Eisen oder mehr äquivalent ist, als Einnahmemenge für einen Tag.

6. α-Glucosidase-inhibierende Komponente zur Verwendung gemäß irgendeinem der Ansprüche 1 bis 5, welche in einem Nahrungsmittel- oder Getränkematerial enthalten ist.


**Revendications**

1. Composant inhibiteur d'α-glucosidase qui est au moins choisi dans le groupe constitué de l'acarbose, du voglibose, du miglitol, d'un produit pulvérisé ou d'un extrait d'un végétal du genre Salacia, de la désoxynojirimycine, d'un polyphénol de la feuille de goyave, d'un extrait de la feuille de murier, d'un extrait de thé vert et d'un extrait de haricot noir fermenté, pour utilisation dans un procédé de prévention ou d'amélioration de l'anémie.

2. Composant inhibiteur d'α-glucosidase pour utilisation selon la revendication 1, dans lequel le composant inhibiteur d'α-glucosidase est au moins choisi dans le groupe constitué du salacinol et du kotalanol.

3. Composant inhibiteur d'α-glucosidase pour utilisation selon la revendication 1 ou la revendication 2, qui accélère l'absorption du fer, du calcium, du magnésium ou du zinc.

4. Composant inhibiteur d'α-glucosidase pour utilisation selon l'une quelconque des revendications 1 à 3, qui présente une activité, en tant que concentration d'inhibition de la sucrase de 50 % (valeur $IC_{50}$) de 0,01 µg/ml ou plus, mais de 800 µg/ml ou moins.

5. Agent pour améliorer l'anémie pour utilisation selon la revendication 4, qui est un agent pour améliorer la carence en fer, l'agent comprenant en outre un composé équivalent à 1 mg ou plus de fer en tant que quantité ingérée pendant un jour.

6. Composant inhibiteur d'α-glucosidase pour utilisation selon l'une quelconque des revendications 1 à 5, qui est contenu dans un aliment ou dans une boisson.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002255832 A
- JP 2008050277 A
- JP 2004154126 B
- JP 2004194635 B
- JP 2007161620 B
- EP 1645557 A

### Non-patent literature cited in the description

- *J. Nutr. Sci. Vitaminol.,* 2003, vol. 49, 7-12
- **MONARREZ- ESPINO.** *J. et.al., FASEB Journal,* 2007, vol. 21, A682
- **FAILLA, M.L.** *Drugs in Development,* 1993, vol. 1, 155-162
- *Bioorganic & Medicinal Chemistry,* 2002, vol. 10, 1547-1554